(19) **Europäisches Patentamt**
**European Patent Office**
**Office européen des brevets**

(11) **EP 4 458 970 A1**

(12) **EUROPEAN PATENT APPLICATION**
published in accordance with Art. 153(4) EPC

(43) Date of publication:
**06.11.2024 Bulletin 2024/45**

(21) Application number: **22916073.4**

(22) Date of filing: **27.12.2022**

(51) International Patent Classification (IPC):
*C12N 15/12* (2006.01)     *C12N 15/31* (2006.01)
*C12P 21/02* (2006.01)     *C07K 14/195* (2006.01)
*C07K 14/37* (2006.01)     *C07K 14/415* (2006.01)
*C07K 14/435* (2006.01)

(52) Cooperative Patent Classification (CPC):
**C07K 14/195; C07K 14/37; C07K 14/415;
C07K 14/435; C12P 21/02**

(86) International application number:
**PCT/JP2022/048103**

(87) International publication number:
**WO 2023/127850 (06.07.2023 Gazette 2023/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.12.2021 JP 2021212750**

(71) Applicant: **Rigaku Corporation
Akishima-shi
Tokyo 196-8666 (JP)**

(72) Inventors:
• **MATSUMOTO Takashi
Akishima-shi, Tokyo 196-8666 (JP)**
• **SATO Takashi
Akishima-shi, Tokyo 196-8666 (JP)**

(74) Representative: **Maiwald GmbH
Elisenhof
Elisenstraße 3
80335 München (DE)**

(54) **MULTI-COMPONENTED STRUCTURE FOR CAPTURING COMPOUND**

(57)     Provided is a structure for capturing a compound that includes at least one gene or a product thereof, or a combination thereof among a group of genes involved in the intracellular and extracellular transport function of the compound, the gene or the product thereof being multi-componented.

EP 4 458 970 A1

**Description**

TECHNICAL FIELD

The present invention relates to a structural body for capturing a compound, which comprises a specific gene selected from a group of genes involved in the intracellular/extracellular transport function of the compound, or a product thereof.

BACKGROUND ART

Structural analysis of molecular structures is important in various fields, such as synthetic chemistry, natural product chemistry, and metabolic analysis. Compounds handled in these fields vary widely, and researchers use different analytical tools, such as mass spectrometry and nuclear magnetic resonance, for their structural analysis. Single-crystal X-ray diffraction method is known as a method for determining the molecular structures of organic compounds, but it is difficult to use single-crystal X-ray diffraction method because a sufficient amount of single crystals cannot be obtained when the organic compounds are small in amount. Therefore, recently, a new technique for single-crystal X-ray diffraction, called "crystalline sponge method", which does not require any crystallization process at all, has been developed (Patent literature 1-3). According to this technique, a single crystal sample for measurement can be prepared simply by soaking a porous crystal called a crystalline sponge in a solution of a compound of interest.

However, the size of molecules that can be analyzed with a crystalline sponge is limited because the target compound needs to be incorporated into its pores.

Meanwhile, some proteins are known to be able to bind to various organic compounds. RamR, a multidrug-resistance regulator protein, is one such protein, and there are examples showing that RamR can be used for the structural analysis of organic compounds (Non-patent literature 1). Since RamR crystals are hydrophilic, a structure determination method using RamR may be useful for the structural analysis of compounds that cannot be handled by the crystalline sponge method.

CITATION LIST

Patent Literature

Patent literature 1: Pamphlet of International Publication WO2011/062260
Patent literature 2: Pamphlet of International Publication WO2014/038220
Patent literature 3: Pamphlet of International Publication WO2014/038221

Non-patent literature

Non-patent literature 1: Takashi Matsumoto et al., Biochemical and Biophysical Research Communications 518 (2019), 402-408

SUMMARY OF INVENTION

Technical Problem

In view of the above background, a capture structural body that can bind to compounds other than RamR is needed to perform structural analysis on a virtually wide variety of compounds.

Solution to Problem

The present invention is as follows.

[1] A structural body for capturing a compound, the structural body comprising at least one gene of a group of genes involved in the intracellular/extracellular transport function of the compound, a product thereof, or a combination thereof, wherein the gene or the product thereof is multi-componented.
[2] The structural body according to [1], wherein the gene, a product thereof, or a combination thereof is labeled.
[3] The structural body according to either one of [1] and [2], wherein the group of genes is a group of genes involved in the regulation of the function or expression of an intracellular/extracellular transport function effector protein.

[4] The structural body according to [3], wherein the transport is intracellular influx or extracellular efflux.

[5] The structural body according to [1], wherein the product is a protein.

[6] The structural body according to [5], wherein the protein is a soluble protein.

[7] The structural body according to either one of [5] and [6], wherein the protein has a specific expression form from among a plurality of expression forms.

[8] A kit for analyzing a compound, the kit comprising the structural body according to any one of [1]-[7].

[9] A method for producing a structural body for capturing a compound, the method comprising the steps of: culturing a transformant containing an expression vector containing at least one gene of a group of genes involved in the intracellular/extracellular transport function of the compound; and collecting an expression product of said gene from the resulting culture product, wherein the gene is a multi-componented gene.

[10] A method for binding a compound to the structural body according to any one of [1]-[7], the method comprising a step of mixing the structural body with the compound.

[11] A method for crystallizing the structural body according to any one of [1]-[7], the method comprising a step of mixing the structural body with a solution for crystallization.

[12] A method for analyzing a structural body crystallized by the method according to [11], the method comprising a step of analyzing the structure of the structural body by molecular replacement method.

Advantageous Effects of Invention

According to the present invention, structural bodies that have affinity for a wide range of compounds can be obtained, and these structural bodies can be used as cages as a substitute for the crystalline sponge method, i.e., as structural bodies for capturing compounds.

BRIEF DESCRIPTION OF DRAWINGS

[Figure 1] A picture of a crystal of a RamR-ethidium complex.

[Figure 2] A picture of a crystal of a RamR-cholic acid complex.

[Figure 3] A view of a multiwell plate in which structural bodies of different designs are pre-arranged on a grid.

[Figure 4] A view showing a well having a structure of a microfluidic channel system or the like with reservoirs arranged in a clover-like pattern.

[Figure 5] A picture of SDS-PAGE showing the result of AcrR purification.

[Figure 6] A picture of a crystal of AcrR.

[Figure 7] A picture of SDS-PAGE showing the results of MarA, EmrA and SoxR expressions.

DESCRIPTION OF EMBODIMENTS

1. Outline

The present invention relates to a structural body for capturing a compound, which comprises a specific gene selected from a group of genes involved in the intracellular/extracellular transport function of the compound, or a product thereof.

The conventional crystalline sponge method utilizes so-called Metal-Organic-Framework (MOF) porous crystals, in which metal ions and organic bridging ligands are assembled via self-assembling processes, as a "cage" for a target analyte.

However, if a cage can be made that is capable of binding to multiple compounds, it does not necessarily have to be a MOF. According to the crystalline sponge method, the difference in the affinity between the crystalline sponges and the target compounds leads to the variety (diversity) of the accommodating conditions, and thus an experiment requires a significant number of repeated condition studies.

Therefore, the present inventors focused on proteins. Some proteins have affinity for various organic compounds such as anionic, cationic, amphoteric and neutral compounds. Therefore, the present inventors set out to establish a method for determining the molecular structure of an organic compound using a protein crystal, thereby completing the present invention.

Thus, the present invention provides a structural body for capturing a compound, which comprises at least one gene of a group of genes involved in the intracellular/extracellular transport function of the compound, a product thereof, or a combination thereof.

2. Structural body for capturing compound

(1) Capture structural body

According to the present invention, a specific gene of a group of genes involved in the intracellular/extracellular transport function of the compound, or a product thereof is used as a structural body for capturing a compound. The transport is related to the intracellular influx or extracellular efflux.

If a gene or a product thereof is involved in the transport function of a compound, it is possible to capture the compound.

(i) Genes involved in intracellular/extracellular transport function of compound

The origin of genes involved in the intracellular/extracellular transport functions of compounds is not particularly limited, and includes animals, insects, plants, yeast, bacteria, archaea, etc.

Examples of substances encoded by the genes involved in the intracellular/extracellular transport functions of compounds include, but are not limited to, membrane transporters that govern the transport (efflux or influx) of substances across biological membranes, pollutant transport factors that bind to persistent organic pollutants and transport them from root to fruit, transcription factors that regulate the expressions of the membrane transporters, transcription factors that regulate the expressions of the transcription factors, and modification enzymes that regulate the expressions of the membrane transporters and the transcription factors.

The group of genes involved in the intracellular/extracellular transport function of a compound includes both genes involved in efflux and genes involved in influx. The genes used in the present invention are not limited as long as they encode a membrane transporter, a protein that regulates the expression of the membrane transporter, and a transport factor that binds to a compound and transfers in vivo.

For example, the above group of genes is a group of genes involved in the regulation of the function or expression of an intracellular/extracellular transport function effector protein. Specifically, such a group of genes include acrR and acrS in Gram-negative bacteria, qacR in Gram-positive bacteria, mlp of the major latex protein family in plants, rahR in the Diapheromeridae family, ABCB1 (MDR1) in humans (animals), and the like.

Various transcription factors (different families) and transport factors exist in various fungi, plants and insects (Table 1), and, for example, these transcription factors can be used in the present invention.

[Table 1]

TABLE 1. Transcriptional regulators that control expression of genes encoding bacterial drug efflux components

| Organism | Regulatory protein(s) | Regulator family[a] | Function of regulator | Ligand(s) of regulatory protein[b] | Drug efflux gene(s) regulated[c] | Reference(s) |
|---|---|---|---|---|---|---|
| RND pump regulators | | | | | | |
| Acinetobacter baumannii | Orf2-Orf3 | Two-component system? | | ? | adeABC* | 111 |
| Burkholderia pseudomallei | AmrR | TetR | Repressor? | ? | amrAB-oprA | 133 |
| Escherichia coli | AcrR | TetR | Repressor | ? | acrAB* | 107 |
| | AcrS | TetR | Repressor? | ? | acrEF* | 156 |
| | BaeR-BaeS | Two-component system | | ? | mdtABC | 12, 137 |
| | EvgA-EvgS | Two-component system | | ? | yhiUV | 144 |
| | MarA/ SoxS/Rob | AraC | Global activators | Rob? (MarA/ SoxS[NA]) | acrAB* and tolC | 69, 70, 118, 177 |
| | MarR | MarR | Repressor of marA | DNP, Pg, Sa, Md | acrAB* and tolC, via MarA | 6, 8, 120 |

(continued)

TABLE 1. Transcriptional regulators that control expression of genes encoding bacterial drug efflux components

| Organism | Regulatory protein(s) | Regulator family[a] | Function of regulator | Ligand(s) of regulatory protein[b] | Drug efflux gene(s) regulated[c] | Reference (s) |
|---|---|---|---|---|---|---|
| Neisseria gonorrhoeae | MtrA | AraC | Global activator | HAs? | mtrCDE* | 184 |
| | MtrR | TetR | Repressor | ? | mtrCDE* and farAB | 106 |
| Pseudomonas aeruginosa | MexR | MarR | Repressor | ? | mexAB-oprM* | 32, 170 |
| | MexT | LysR | Activator | ? | mexEF-oprN* | 81 |
| | MexZ | TetR | Repressor? | ? | metAY | 4 |
| | NfxB | LacI/GalR | Repressor | ? | mexCD-oprJ* | 169 |
| Pseudomonas putida | ArpR | TetR | Repressor? | ? | arpABC | 78 |
| Stenotrophomonas maltophilia | SmeR-SmeS | Two-component system? | | ? | smeABC | 100 |
| MFS pump regulators | | | | | | |
| Bacillus subtilis | BltR | MerR | Activator | ? | blt | 3 |
| | BmrR | MerR | Activator | R6G, TPP, Ao, DEC, ABM, ADCP, DDPB | bmr | 2, 227, 244, 245 |
| | Mta | MerR | Global activator | ? | bmr and blt | 13, 43 |
| Escherichia coli | EmrR | MarR | Repressor | CCCP, DNP, Eb, FCCP, Na, Sa, TCS | emrAB* and mcbABCDEFG | 20, 104, 105, 238 |
| | EvgA-EvgS | Two-component system | | ? | emrKY | 145 |
| | TetR | TetR | Repressor | Tc | tetA | 59, 60, 153 |
| Staphylococcus aureus | ArlR-AlrS | Two-component system | | ? | norA*, via 18-kDa protein | 35 |
| | QacR | TetR | Repressor | Be, Be, Ch, Cv, Dc, Eb, Mg, Pf, R6G | qacA/qacB | 47, 48, 197, 198 |

Here, in general, the affinity of a compound for a protein is specific in a one-to-one manner. However, a certain group of transcription factors, effector proteins, and the like have the unique property of having binding affinity for multiple compounds. According to the present invention, the properties of such proteins are used to capture compounds.

In addition, examples of factors that regulate the expressions of multidrug efflux proteins from pathogenic bacteria include the following factors (Table 2).

[Table 2]

- AcrR : TetR family transcriptional repressor
- AcrS/EnvR : TetR family transcriptional repressor
- EmrR . MarR family transcriptional repressor
- MarR . MarR family
- MarA . AraC family global transcriptional regulator

(continued)

- SoxS . AraC family global transcriptional regulator
- Rob . AraC family global transcriptional regulator
- MexR : MarR family transcriptional repressor
- MexT . LysR family transcriptional repressor
- MexZ : TetR family transcriptional repressor
- QacR : TetR family transcriptional repressor
- RamR/A : TetR family
- SoxR : MerR family

In addition, examples of the factors used in the present invention include the followings.

AcrR (TetR family) from *E. coli*
AcrS (TetR family) from *E. coli*
QacR (TetR family) from *Staphylococcus aureus*
ToxT (AraC family) from *Vibrio cholerae* O395
PqsR (LysR family) from *Pseudomonas aeruginosa*
Ginseng major latex-like protein 151 (GLP: MLP family) from the plant Asian ginseng
RhaR (AraC family) from Bacteria Latreille et al. 1825 of the insect belonging to the Diapheromerini tribe of the Diapheromeridae family
Multidrug-resistance regulator protein MarA from *E. coli*
Multidrug-resistance regulator protein EmrA from *E. coli*
Multidrug-resistance regulator protein SoxR (MerR family) from *E. coli*

(ii) Products of genes involved in transport function of compound

Products of genes refer to RNAs, proteins, and complexes thereof resulting from the expressions of the genes, and include transcription products and expression products. Transcription generally refers to the synthesis of RNA based on the nucleotide sequence of DNA of a chromosome or organelle, and a transcription product refers to the synthesized RNA.

Moreover, an expression product refers to a protein translated from RNA. Herein, proteins include both peptides and polypeptides. In addition, proteins may be insoluble (membrane proteins) or soluble proteins.

The present invention further comprises a combination of a transcription product and an expression product.

Examples of the combination of a gene and a product thereof include a helicase and DNA, histones and DNA, and a transcription factor and a target gene.

- Helicase binds to DNA and separates double strand into single strands.
- Histones form a nucleosome which consists of DNA wrapping around histones.
- Transcription factor protein binds to a target gene sequence and regulates the expression of a downstream protein. When a compound can be captured and analyzed in the present invention, the transcription factor that captured the compound forms a complex with DNA.

(iii) Expression forms (diversity of products)

Gene expression products include post-translationally modified forms resulting from biological processes or chemical reactions in the broadest sense. Examples of such post-translationally modified forms include: those that affect a peptide chain, such as a peptide strand break and disulfide bond formation; modified structures resulting from deamination, deamidation, and simultaneously occurring β-amino acid modification, and oxidative/reductive reactions; and phosphorylated, alkylated, acylated, glycosylated, and lipid modified structures that undergo biological processes.

In the present invention, such a modified form can also be used.

The term "expression form" herein include, for example, a different product that resembles the protein of interest but presents a different form (multiple expression forms) due to post-translational modification (chemical or non-biological modification). Therefore, according to the present invention, it is possible to select a product without such a modification (one having the specific expression form) or a product with such a modification.

(2) Production of product of gene

According to the present invention, a genetic engineering method can be employed to obtain a product from a gene.

First, to produce a product of a gene from a natural product, the gene is isolated using a commercially available gene extraction kit. Examples of the gene extraction kit include DNA or RNA extraction kits of the ISOGEN series and ISOSPIN series (Nippon Gene Co., Ltd.). Direct polymerase chain reaction (PCR), which amplifies the target DNA directly from the sample, can also be used.

If the nucleotide sequence of the gene is known, it can be easily obtained by chemical synthesis or by reaction using PCR.

Once the DNA of interest is obtained, the DNA is linked to (inserted into) an appropriate vector to obtain a recombinant vector. A method for obtaining a recombinant vector is known (Sambrook J. et al., Molecular Cloning, A Laboratory Manual (4th edition), Cold Spring Harbor Laboratory Press (2012)). A vector for inserting the DNA of the present invention is not particularly limited as long as it can replicate in the host, and examples include plasmid DNAs, phage DNAs, and viruses.

Examples of the plasmid DNAs include plasmids from *E. coli,* plasmids from *Bacillus subtilis,* and plasmids from yeast, and examples of phage DNAs include lambda phages. Moreover, examples of viruses include adenoviruses and retroviruses.

According to the present invention, if desired, a cis-element such as an enhancer, a splicing signal, a poly-A addition signal, a ribosome-binding sequence (SD sequence), a selection marker gene, a reporter gene, and the like, in addition to the promoter and the DNA, can also be linked to the recombinant vector. Examples of the selection marker gene include dihydrofolate reductase gene, ampicillin-resistance gene, neomycin-resistance gene, etc. Examples of the reporter gene include green fluorescent protein (GFP) and mutants thereof (fluorescent proteins such as EGFP, BFP, and YFP), luciferase, alkaline phosphatase, and genes such as LacZ.

According to the present invention, the above recombinant vector of the present invention is introduced into a host such that the gene of interest can be expressed to obtain a transformant.

The host used for the transformation is not particularly limited as long as it is capable of expressing the gene of interest. Examples of the host include bacteria (*E. coli, Bacillus subtilis,* etc.), yeast, animal cells (COS cells, CHO cells, etc.), insect cells, and insects. A mammal such as a goat or a plant such as lettuce or tobacco can also be used as the host. A method for introducing a recombinant vector into a host is known (Sambrook J. et al., Molecular Cloning, A Laboratory Manual (4th edition), Cold Spring Harbor Laboratory Press (2012)).

When a bacterium is used as the host, the recombinant vector of the present invention should be capable of autonomous replication in said bacterium and capable of containing a promoter, a ribosome-binding sequence, the DNA of interest, and a transcription termination sequence. Examples of the bacterium include *E. coli (Escherichia coli).* The promoter may be, for example, lac promoter. There are various known methods for introducing a vector into a bacterium, such as calcium ion method.

When yeast is used as the host, for example, *Saccharomyces cerevisiae* or the like can be used. In this case, the promoter is not particularly limited as long as it can be expressed in yeast, and, for example, it may be gall promoter or the like. Examples of the method for introducing a vector into yeast include electroporation method and spheroplast method.

Then, the transformant is cultured and the protein of interest is harvested from the culture product. The term "culture product" may refer to (a) a culture supernatant, or (b) any of cultured cells, cultured bacteria, or a crude product thereof.

If the protein of interest is produced inside the bacteria or cells after the culture, the protein is extracted by disrupting the bacteria or cells. If the protein of interest is produced outside of the bacteria or cells, the culture solution is used as it is or the bacteria or cells are removed by centrifugation. Then, a general biochemical method used for protein isolation and purification, such as ammonium sulfate precipitation, gel filtration, ion exchange chromatography, affinity chromatography, hydrophobic interaction chromatography, reversed-phase chromatography, or the like, can be used alone or in combination as appropriate to isolate and purify the protein of interest.

Here, once the protein of interest is isolated, it is preferable to determine its amino acid sequence. For the structural analysis of the protein of the present invention, the purified protein component is analyzed for its amino acid sequence structure using a protein sequencer or a mass spectrometer, or a combination of these two instruments, depending on the amount existing.

The protein used in the present invention has a predetermined amino acid sequence, but other than this amino acid sequence, any amino acid sequence which has one or more amino acids deleted, substituted or added in said amino acid sequence can be used as the structural body of the present invention as long as it has the functions of the original protein.

Moreover, when the protein of the present invention is expressed from a gene, other than the nucleotide sequence of this gene itself, DNA that hybridizes with DNA comprising a nucleotide sequence complementary to said nucleotide sequence under stringent conditions and that encode an amino acid sequence of a protein having the activity of the protein of interest can also be used.

Herein, "stringent conditions" may be any of lowly, moderately or highly stringent conditions, and "lowly stringent conditions" refer to conditions of, for example, 5x SSC, 5x Denhardt's solution, 0.5% SDS, and 50% formamide at 32°C. The "moderately stringent conditions" refer to conditions of, for example, 5x SSC, 5x Denhardt's solution, 0.5% SDS, and 50% formamide at 42°C. The "highly stringent conditions" refer to conditions of, for example, 5x SSC, 5x Denhardt's solution, 0.5% SDS, and 50% formamide at 50°C. Under these conditions, highly homologous DNA is expected to be obtained more efficiently at higher temperatures. Note that multiple factors including the temperature, the probe concentration, the probe length, the ionic strength, the time, the salt concentration, and the like are considered to affect the stringency of hybridization, but those skilled in the art can appropriately select these factors to achieve similar stringency.

In addition to the above, examples of hybridizable DNAs include DNAs that have approximately 70% or more homology (identity) and further DNAs that have approximately 80% or more, 85% or more, 90% or more, 95% or more, 96% or more, 97% or more, 98% or more, 99% or more homology (identity) with the DNA encoding the protein of interest, when calculated by a homology search software such as FASTA and BLAST using default parameters.

(3) Multi-component of gene product

When affinity of a protein for a compound is too high, it is likely to bind to the compound of interest and also to substances other than the compound of interest. Therefore, if the full-length sequence of the protein is used for the gene expression and purification, substances other than the compound of interest are bound during the expression and purification stages. For this reason, it is preferable to inactivate it so that the affinity does not increase during the above expression and purification stages.

Thus, according to the present invention, the gene is divided into several fragments and componented (modularized) so that each component is expressed separately. The method for the expression of each component is the same as that described above. Then, after purification, the components are mixed to make a complete structural body, which is allowed to bind to a compound.

Here, the components may be divided equally, or the ratio of division (ratio of the lengths of the gene fragments) may be changed.

For example, if a component is equally divided into fragments 1 and 2, the structural body of the present invention takes on a form in which the compound of interest would be sandwiched between fragments 1 and 2. Alternatively, if the lengths of the fragments are changed, the structural body may take on a form in which it is divided into a pocket region, into which the compound of interest enters, and a lid region, which covers the pocket. In this case, the lid region may be one, or the fragment may be divided into a plurality of lids. In addition, the compound of interest does not necessarily bind to all of the divided fragments, and it is also possible for other component to induce the binding of the compound of interest to the binding component.

Furthermore, according to the present invention, whether or not the multi-componented product has successfully captured the compound of interest can be visualized by labeling at least one component. For example, a donor is added to one component and an acceptor is added to the other, so that the donor and the acceptor produce fluorescence or luminescence because they are in close proximity to each other when a componented product is formed as a whole. As a result, a product that has captured the compound of interest can be obtained by visible or spectroscopic observation.

If a luminescent protein is used for visualization, the structural body can be divided into two components and each component can be fused with the N-terminal and C-terminal portions of the divided luminescent protein. A similar technique can also be used for a fluorescently-labeled compound, but the portion to be introduced is not limited to the N-terminal and C-terminal portions and it can be introduced into a portion having an appropriate functional group. In this case, a linker can be designed to exist between the product and the labeled compound.

Furthermore, in the case where phosphorescence is used, a technique similar to those used for a luminescent protein or a fluorescently-labeled compound can be used.

A His-tag can also be used. In this case, a component with a long His-tag and a component with a short His-tag are designed so that only the structural body that captured the compound of interest can be separated and collected by utilizing the affinity for an immobilized metal.

(4) Compound

According to the present invention, a "compound" includes a substance formed by the binding of two or more types of elements through a chemical reaction, i.e., a molecule consisting of two or more types of elements linked by chemical bonds, as well as a molecule consisting of a single element but having a specific structure consisting of a plurality of atoms. Although the "compound" may be an organic or inorganic molecule, in one aspect, it is an organic molecule.

According to the present invention, a compound to be captured is a target of measurement, and includes any of aromatic hydrocarbon compounds, aliphatic hydrocarbon compounds, alicyclic hydrocarbon compounds, etc., as well as biopolymers such as amino acid derivatives including amino acids, peptides, polypeptides, and oligonucleic acids, and compounds having a specific structure that does not contain a carbon atom, such as S6. The compound may be a saturated compound or an unsaturated compound. When the compound has a structure or property that is recognized and bound by a capture structural body, it is captured due to its affinity for the product. Therefore, the type of the compound is not limited as long as the compound has such a structure or property, and any such compounds can be used as the target of capture.

In one aspect of the present invention, the aromatic hydrocarbon compounds are a group of unsaturated cyclic organic compounds with 3-28 carbon atoms, which includes aromatic hydrocarbons and heteroaromatic compounds.

Aliphatic hydrocarbons are not particularly limited as long as they can be captured by the capture structural body of the present invention, and examples thereof include saturated aliphatic hydrocarbons with 2-30 carbon atoms in a chain, branched or cyclic form, or unsaturated aliphatic hydrocarbons with 2-24 carbon atoms in a chain or branched form, having one or two or more double or triple bonds in the molecule.

Examples of the alicyclic hydrocarbons include saturated alicyclic hydrocarbons with 3-37 carbon atoms, or unsaturated alicyclic hydrocarbons with 3-37 carbon atoms, having one or two or more double or triple bonds in the molecule thereof.

Furthermore, in the present invention, the compounds to be captured include natural products, fat and oil compounds (fats), sugar compounds (carbohydrates), peptides, nucleic acid compounds (DNAs or RNAs), alkaloid compounds, steroid compounds (terpene compounds), biological substances, high-molecular-weight compounds, and dendrimers.

Specifically, the technique of the present invention can be applied to structural analysis of a group of various compounds derived from various drug metabolites having complex structures, which are generated *in vivo* after administration, and environmentally hazardous substances such as POPs, PFOS, and PFOA, which have been difficult to be analyzed by conventional structural analysis methods.

3. Kit

The present invention provides a kit for analyzing a compound, the kit comprising the aforementioned structural body.

The kit of the present invention may include, in addition to the structural body of the present invention, but not limited to, various buffers, a crystallization solution, a crystallization container, sterile water, an operation manual (instructions).

If the structural body is a multi-componented structural body, each protein can be housed separately in its own container. The crystallization container is based on the sitting drop technique, but can also have a form adapted to a microplate reader for rapid screening. In a fluorescence observation kit, the crystallization plate may be opaque (black) so that fluorescence, phosphorescence, or luminescence observation can be performed simultaneously.

Furthermore, in the kit of the present invention, the container for placing the structural body may be in the form of a multiwell plate in which crystallization is performed. In this case, each kit may contain only one structural body or may contain multiple structural bodies. In a case where multiple structural bodies are included, for example, the structural bodies are placed in the respective wells of the multiwell plate.

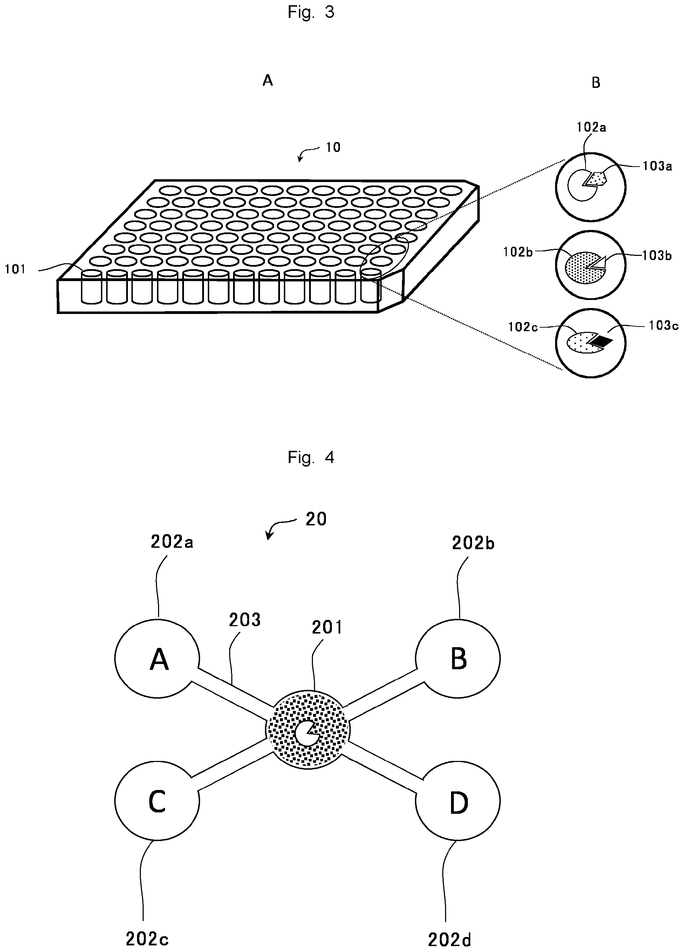

In a multiwell plate provided (Figure 3), structural bodies of different designs are arranged on the grid in advance, and a compound of interest is added to each grid cell to enable comprehensive binding operations. Among the different structural bodies, one can easily find a structural body that crystallizes with the compound of interest.

Figure 3A shows a multiwell plate 10 with a predetermined number of wells 101. Figure 3B is a view showing an aspect in which structural bodies are contained in three wells. Figure 3B shows an aspect in which the three wells contain different structural bodies 102a, 102b, and 102c that bind to compounds of interest 103a, 103b, and 103c, respectively.

Each single well of the above multiwell plate may have not only a simple reservoir structure but also a structure such as a microfluidic channel system (Figure 4). Figure 4 shows a structure 20 of a microfluidic channel system. For example, in Figure 4, solution reservoirs 202a, 202b, 202c, and 202d arranged in a clover shape are placed in each well with a mixing reservoir 201 provided in the center, such that each solution reservoir 202 is connected to the mixing reservoir 201 via a microfluidic channel 203. The reservoirs 202a, 202b, 202c, and 202d contain solution A, solution B, solution C, and solution D, respectively, and by passing each solution from each solution reservoir 202 to the mixing reservoir 201, mixing of the solutions and crystallization can be performed simultaneously and efficiently. In addition, the microfluidic channel system can be filled with a gel-like substance to further improve the success rate of crystallization. The above microfluidic channel system may exist not only as a single well in a multiwell plate but also in an independent form. Furthermore, the number of reservoirs and the shape of their arrangement are optional and not particularly limited.

4. Method for binding compound to structural body

The present invention provides a method for binding a compound to the above structural body, the method comprising a step of mixing the structural body with the compound.

Once mixed, the compound binds to the structural body according to the affinity of the structural body. The mode of binding depends on the type of the structural body and the type of the compound of interest.

The binding to the compound can be easily confirmed by spectroscopic analysis using, for example, fluorescence, phosphorescence, or luminescence as described above, and the solution is preferably used for the subsequent crystallization. Note it is also possible to mix the structural body and the compound of interest under crystallization conditions to allow them to crystallize as they are. In doing so, crystals of structural bodies that did not incorporate the compound of interest may precipitate, but these crystals can be easily distinguished because they do not present fluorescence, phosphorescence, or luminescence.

5. Method for crystallizing structural body

The present invention provides a method for crystallizing the above structural body.

Examples of the method for crystallizing a structural body include, but are not limited to, hanging drop vapor diffusion method, sitting drop vapor diffusion method, bulk batch method, micro batch method, free surface diffusion method, and microdialysis method.

In one aspect of the present invention, crystallization can be performed, for example, by vapor diffusion method.

6. Method for analyzing structure of structural body

The present invention provides a method for analyzing the structure of the aforementioned crystallized structural body.

Steps for analyzing the structure of a crystallized protein are not particularly limited. For example, it is possible to employ not only a method of collecting diffraction data using, for example, the high flux beamline (BL40XU) at the large synchrotron radiation facility (SPring-8), etc., but also a method of collecting high-precision diffraction data using a laboratory X-ray structural diffractometer (XtaLab Synergy system). Since the structures of the structural bodies are known, it is common to use a method in which they are used as search models to determine the initial phase by molecular replacement method to determine the initial structures before obtaining the final structures. In the case of high-precision diffraction data collected using a laboratory X-ray diffractometer, *ab initio* phase determination method such as S-SAD can be employed.

For the structural analysis by molecular replacement method, prescribed program packages, such as CCP4 (Collaborative computational project, number 4) program package, have been distributed.

EXAMPLES

Hereinafter, the present invention will be described further in detail by way of examples. The scope of the present invention, however, should not be limited to these examples.

Example 1

Expression, purification, crystallization, measurement, and structural analysis of RamR

- The ramR gene was amplified from the chromosomal DNA of strain: *Salmonella enterica* serovar Typhimurium by PCR and inserted into pETDuet-1 to obtain plasmid pETDuet-lramR.

Culture of *E. coli* and expression of RamR

- *Salmonella enterica* serovar Typhimurium-derived RamR was expressed using *E. coli* C41 (DE3) transformed with pETDuet-lramR. Hereinafter, *E. coli* refers to C41(DE3)/pETDuet-1ramR.

- A frozen stock of *E. coli* was inoculated into 10 mL of a LB medium (pre-pre-culture) containing ampicillin at a concentration of 100 $\mu$g/mL and then cultured at 37°C.
- After overnight culture, to a LB medium for pre-culture (containing ampicillin at a concentration of 100 $\mu$g/mL), the pre-pre-culture medium was added at 1/10 volume of the LB medium for pre-culture and cultured at 37°C.
- After 2 hours of culture, to a LB medium for main culture (containing ampicillin at a concentration of 100 $\mu$g/mL), the medium for pre-culture was added at 1/100 volume of the LB medium for main culture and cultured at 37°C.
- After culturing to Optical Density: $OD_{600}$ = 0.6-0.7, IPTG (isopropyl $\beta$-D-thiogalactopyranoside) was added to a final concentration of 0.1 mM and induction culture was performed for 3 hours.
- After the induction period, cells were collected by high-speed centrifugation (6,800 x g, 10 min., 4°C).
- *E. coli* pellets were suspended in washing buffer (50 mM Tris-HCl (pH 7.0), 1 mM $MgCl_2$, 0.5 mM EDTA (Ethylenediaminetetraacetic acid), 10% Glycerol), and then subjected to high-speed centrifugation (8,000 x g, 10 min., 4°C) to obtain the washed *E. coli* pellets.
- The resulting *E. coli* pellets were frozen using liquid nitrogen and stored in an ultra-low temperature freezer at -80°C.

Purification of RamR

- After the frozen *E. coli* pellets were suspended using buffer A (50 mM sodium phosphate (pH7.0), 1 mM EDTA, 1 mM DTT (dithiothreitol)), protease inhibitor cocktail was added, and *E. coli* were disrupted using French press.
- After the disruption, the supernatant resulting from high-speed centrifugation (9,700 x g, 15 min., 4°C) was separated to remove undisrupted cells and insoluble substances.
- The obtained supernatant was further ultracentrifuged (229,000 x g, 60 min., 4°C) to obtain a supernatant without the membrane fraction.
- The resulting supernatant was applied to SP-Sepharose FF (cation exchanger) equilibrated with buffer A, then washed with buffer A, and eluted with a gradient of NaCl at concentrations from 0 to 500 mM.
- After confirming the degree of purification of the eluted fractions by SDS-PAGE, the fraction containing RamR was concentrated by ultrafiltration.
- The concentrated sample was purified by gel filtration chromatography (Superdex 200 10/300 GL) using buffer B (20 mM sodium phosphate (pH 7.0), 75 mM NaCl, 2 mM DTT).
- After confirming the degree of purification of the eluted fractions by SDS-PAGE, the fraction containing highly pure RamR was concentrated, then diluted and mixed with buffer B, and reconcentrated.
- The buffer solution was homogenized by repeating addition of buffer B and re-concentration several times.
- Concentration was performed to a final protein concentration of 13-40 mg/mL. The protein concentration was determined by protein quantification method.
- The concentrated RamR sample was frozen in liquid nitrogen and then stored in an ultra-low temperature freezer at -80°C or in liquid nitrogen until crystallization.

Crystallization

- A compound to be analyzed was dissolved using ultrapure water, DMSO (dimethyl sulfoxide), or ethanol to prepare a compound stock solution.
- The compound stock solution was added to the highly concentrated RamR solution such that the molar concentration of each compound was 10 to 0.1 times that of RamR, to prepare and mix a mixture to a final protein concentration of 20-10 mg/mL. The mixture was then allowed to stand still for several hours.
- The temperature at which the mixture was allowed to stand still was in the range of 4°C to 25°C.

For example, the temperature was 4°C for ethidium and cholic acid, and 25°C for synthetic gefitinib intermediates.

- The RamR-compound mixture was mixed with a crystallization reservoir solution, and crystallization was performed by vapor diffusion method. Crystallization was conducted at the temperature of 25°C.
- The following conditions were used for the crystallization reservoir.

  10%-15% (w/v) polyethylene glycol (PEG) 3350,
  0.2 M ammonium sulfate,

0.1 M sodium citrate (pH 5.6),
15%-20% (w/v) PEG3350,
0.2 M sodium acetate,
0.1 M 2-morpholinoethanesulfonic acid monohydrate (MES)-Na (pH 6.5)

- Crystal growth was confirmed a few days after the crystallization set-up.

Measurement

- The precipitated crystals were soaked in a cryoprotectant solution (solution obtained by adding 10-20% ethylene glycol to the reservoir solution), and then the crystals were scooped out with a loop and rapidly frozen at liquid nitrogen temperature.
- Frozen crystals were mounted on a single-crystal diffraction system: XtaLAB Synergy system, and then crystal screening was performed.
- Measurement strategy was generated based on the results of the screening measurement, and the main measurement was performed.
- The measurement temperature was 100 K.

Data processing and structural analysis

- CrysAlis[Pro] was used for the data processing, and CCP4 (Collaborative computational project, number 4) program package was used for the structural analysis.
- Molecular replacement method, in which the structure of a protein of interest is analyzed using a structure of a similar protein, was employed for the structural analysis. A structure of a similar protein was downloaded from PDB (Protein Data Bank).
- Since the structure had already been analyzed for RamR, the structural analysis by molecular replacement method was performed using the structure of RamR itself.
- The three-dimensional coordinates of the compound were generated using ChemDraw/Chem3D, and the information file for CCP4 (compositional formula, interatomic distances, and coordinates) was created using CCP4/sketcher.
- The structure obtained by molecular replacement method using CCP4/Molrep was subjected to refinement using CCP4/Refmac5 and model modification using Coot several times.
- Electron density map (structural analysis data) of the compound captured by RamR was obtained after the refinement.
- The generated coordinates of the compound were fitted to the electron density map using Coot, and then subjected to refinement using CCP4/Refmac5 and model modification using Coot several times, thereby obtaining the three-dimensional structure of the compound of interest.

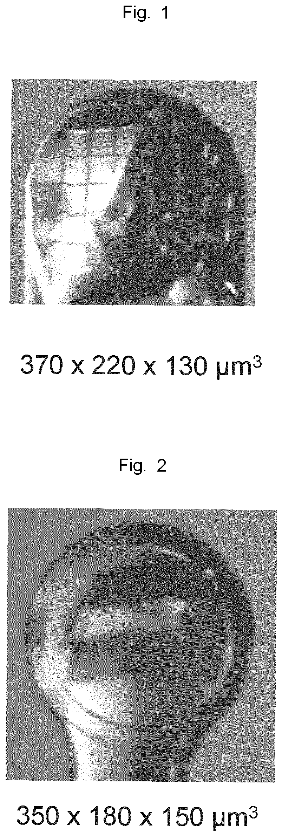

The results are shown in Figures 1 and 2.
Figure 1 is a picture of a crystal of a RamR-ethidium complex, and Figure 2 is a picture of a crystal of a RamR-cholic acid complex. The values below each view indicate the size of the crystal. The lattice constants, Rint, and R-factor of each crystal are shown below.

<Regarding Figure 1>

-Measurement results

Space group: P1
Lattice constants
a = 44.16Å, b = 54.28Å, c = 91.41Å, $\alpha$ = 103.41°, $\beta$ = 97.45°, $\gamma$ = 90.14°
Resolution: 1.70Å
Rint = 7.2%

-Results of structural analysis

R-factor = 16.85%
R-free = 23.48%

<Regarding Figure 2>

-Measurement results

Space group: C2
Lattice constants
a = 86.69Å, b = 53.90Å, c = 44.22Å, $\alpha$ = 90.0°, $\beta$ = 93.06°, $\gamma$ = 90.0°
Resolution: 1.55Å
Rint = 3.1%

-Results of structural analysis

R-factor = 14.61%
R-free = 20.38%

Note that since the binding affinity of RamR for compounds is weaker than that of other transcription factors due to its nature, the possibility of binding to a target compound to allow its structural analysis is not so high. On the other hand, AcrR from *E. coli,* for example, has a large compound-binding pocket and has binding affinity for a wide range of compounds. Thus, use of transcription factors that have high binding affinity and that are always bound to something can be applied to structural analysis of a wider range of compounds. In addition, transcription factors other than those of the TetR family give structural bodies with a widely different structures and different compound affinity. Use of such transcription factors of different families can be applied to the structural analysis of a group of compounds with widely different properties.

Example 2

Expression, purification, and crystallization of *E.* coli-derived multidrug-resistance regulator protein: AcrR

- The acrR gene was amplified from the chromosomal DNA of strain: *Escherichia coli* K12 JM109 by PCR. Primers were designed and used to introduce a histidine tag (6 histidine residues) into AcrR during PCR amplification for the purpose of simplifying the purification process. The plasmid incorporating the amplified acrR into pET-22b(+) was designated pET22b-acrR.

Culture of *E. coli* and expression of AcrR

- *Escherichia coli* K12 JM109-derived AcrR was expressed using *E. coli* C41 (DE3) transformed with pET22b-acrR. Hereinafter, *E. coli* refers to C41(DE3)/pET22b-acrR.
- A frozen stock of *E. coli* was inoculated into 10 mL of a LB medium (pre-pre-culture) containing ampicillin at a concentration of 100 $\mu$g/mL and then cultured at 37°C.
- After overnight culture, to a LB medium for pre-culture (containing ampicillin at a concentration of 100 $\mu$g/mL), the pre-pre-culture medium was added at 1/20 volume of the 2xYT medium for pre-culture and cultured at 37°C.
- After 2 hours of culture, to a 2xYT medium for main culture (containing ampicillin at a concentration of 100 $\mu$g/mL), the medium for pre-culture was added at 1/25 volume of the 2xYT medium for main culture and cultured at 37°C.
- After culturing to Optical Density: $OD_{600}$ = approx. 0.6, the culture solution was cooled, IPTG (isopropyl $\beta$-D-thioga-lactopyranoside) was added to a final concentration of 0.1 mM, and induction culture was performed at 25°C for 10-15 hours.

- After the induction period, cells were collected by high-speed centrifugation (6,800 x g, 10 min., 4°C).
- *E. coli* pellets were suspended in washing buffer (50 mM Tris-HCl (pH 7.0)) and then subjected to high-speed centrifugation (10,000 x g, 5 min., 4°C) to obtain the washed *E. coli* pellets.
- The resulting *E. coli* pellets were stored in an ultra-low temperature freezer at - 80°C.

Purification of AcrR

- After the frozen *E. coli* pellets were suspended using buffer A (20 mM Tris-HCl (pH7.5), 300 mM NaCl, 2 mM 3-me (3-Mercapto-1,2-propanediol)), *E. coli* were disrupted using an ultrasonic disruptor.
- After the disruption, the supernatant resulting from high-speed centrifugation (14,000 x g, 20 min., 4°C) was separated to remove undisrupted cells and insoluble substances.
- The resulting supernatant was further subjected to high-speed centrifugation (14,000 x g, 20 min., 4°C) to obtain a supernatant without insoluble substances.
- The resulting supernatant was applied to Ni-IMAC Sepharose 6 FF equilibrated with buffer A (affinity chromatography), washed with buffer A and imidazole at concentrations of 40 and 100 mM, and then eluted with imidazole at a concentration of 300 mM.
- After confirming the degree of purification of the eluted fractions by SDS-PAGE, the fraction containing highly pure AcrR was concentrated, then diluted and mixed with buffer B (20 mM HEPES (pH7.5), 200 mM NaCl, 60 mM imidazole-HCl (pH 7.5), 2 mM DTT (dithiothreitol)), and reconcentrated.
- The buffer solution was homogenized by repeating addition of buffer B and re-concentration several times.
- Concentration was performed to a final protein concentration of 20-150 mg/mL. The protein concentration was determined by protein quantification method.
- The concentrated AcrR sample was stored in an ultra-low temperature freezer at -80°C or in liquid nitrogen until crystallization.

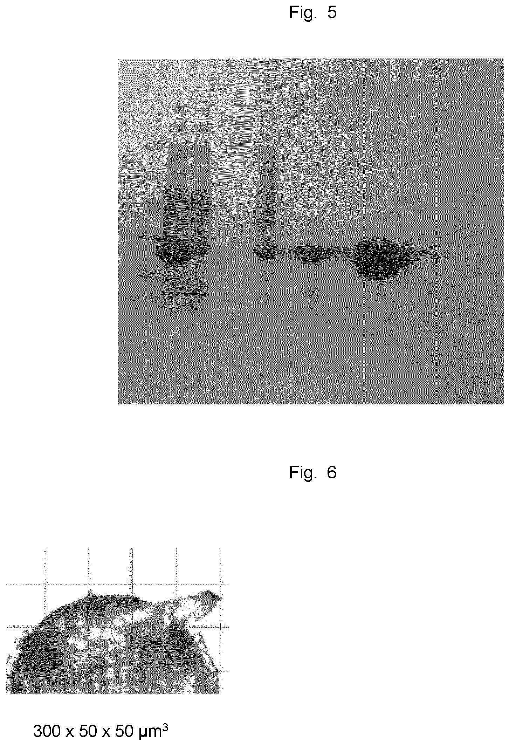

The result of AcrR purification is shown in Figure 5.

Crystallization of AcrR

- AcrR was mixed with a crystallization reservoir solution, and crystallization was performed by vapor diffusion method. Crystallization was conducted at the temperature of 20°C.
- The following conditions were used for the crystallization reservoir.

   25% (w/v) polyethylene glycol (PEG) 3350,
   0.2 M sodium chloride,
   0.1 M Bis-Tris (pH 5.5)

- Crystal growth was confirmed a few days after the crystallization set-up.

Diffraction data acquisition, data processing, and structural analysis

- CrysAlis[Pro] was used for the diffraction data acquisition and the data processing, and CCP4 (Collaborative computational project, number 4) program package was used for the structural analysis.
- Molecular replacement method, in which the structure of a protein of interest is analyzed using a structure of a similar protein, was employed for the structural analysis. A structure of a similar protein was downloaded from PDB (Protein Data Bank).
- Since the structure had already been analyzed for AcrR, the structural analysis by molecular replacement method was performed using the structure of AcrR itself.
- The structure obtained by molecular replacement method using CCP4/Molrep was subjected to refinement using CCP4/Refmac5 and model modification using Coot several times.
- Electron density map of AcrR was obtained after the refinement.

- The result suggested that the structure of AcrR with no compound bound thereto was obtained since there was no residual electron density at the compound binding site.
- It was also suggested that AcrR could be used as a structural body for capturing a compound.

The result is shown in Figure 6.

Figure 6 is a picture of an AcrR crystal. The values below the view indicate the size of the crystal. The lattice constants of the crystal are shown below.

-Measurement results

Space group: I23
Lattice constants

$$a = b = c = 141.34\text{Å}, \alpha = \beta = \gamma = 90.00°$$

Example 3

Expressions, purification, and crystallization of *E.* coli-derived multidrug-resistance regulator proteins: MarA, EmrA, and SoxR

- The MarA, emrA, and soxR genes were amplified from the chromosomal DNA of strain: *Escherichia coli* K12 JM109 by PCR. Primers were designed and used to introduce a histidine tag (6 histidine residues) into each soft protein during PCR amplification for the purpose of simplifying the purification process. The plasmids incorporating the amplified marA, emrA, and soxR into pET-22b(+) were designated pET22b-marA, pET22b-emrA, and pET22b-soxR, respectively.

Culture of *E. coli* and expressions of MarA, EmrA, and SoxR

- *Escherichia coli* K12 JM109-derived MarA, EmrA, and SoxR were expressed using *E. coli* C41 (DE3) transformed with pET22b-marA, pET22b-emrA, and pET22b-soxR, respectively. Hereinafter, *E. coli* refers to C41(DE3)/pET22b-marA, C41(DE3)/pET22b-emrA, or C41(DE3)/pET22b-soxR.
- A frozen stock of *E. coli* was inoculated into 3 mL of a LB medium (pre-culture) containing ampicillin at a concentration of 100 μg/mL and then cultured at 37°C.
- After overnight culture, to a LB medium for culture (containing ampicillin at a concentration of 100 μg/mL), the pre-pre-culture medium was added at 1/20 volume of the 2xYT medium for culture and cultured at 37°C.
- After culturing to Optical Density: $OD_{600}$ = approx. 0.6, the culture solution was cooled, IPTG (isopropyl β-D-thiogalactopyranoside) was added to a final concentration of 0.1 mM, and induction culture was performed at 25°C for 10-20 hours.
- After the induction period, cells were collected by high-speed centrifugation (6,800 x g, 10 min., 4°C).
- *E. coli* pellets were suspended in washing buffer (50 mM Tris-HCl (pH 7.0)) and then subjected to high-speed centrifugation (10,000 x g, 5 min., 4°C) to obtain the washed *E. coli* pellets.
- The resulting *E. coli* pellets were stored in an ultra-low temperature freezer at - 80°C.

Confirmation of MarA, EmrA, and SoxR expressions

- After the frozen *E. coli* pellets were suspended using buffer A (20 mM Tris-HCl (pH7.5), 300 mM NaCl, 2 mM 3-me (3-Mercapto-1,2-propanediol)), *E. coli* were disrupted using an ultrasonic disruptor.
- After the disruption, the supernatant resulting from high-speed centrifugation (14,000 x g, 20 min., 4°C) was separated to remove undisrupted cells and insoluble substances.
- The resulting supernatant was further subjected to high-speed centrifugation (14,000 x g, 20 min., 4°C) to obtain a supernatant without insoluble substances.
- SDS-PAGE (SDS Polyacrylamide gel electrophoresis) was performed to confirm expressions.

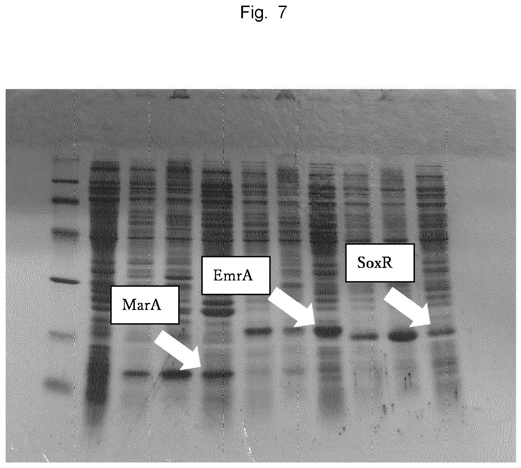

The results are shown in Figure 7. In Figure 7, the bands indicated by the arrows show the bands of MarA, EmrA, and SoxR, confirming expressions for all proteins.

REFERENCE SIGNS LIST

10: Multiwell plate
101: Well
102: Structural body
103: Compound of interest
20: Structure of microfluidic channel system
201: Mixing reservoir
202: Solution reservoir
203: Microfluidic channel

**Claims**

1. A structural body for capturing a compound, the structural body comprising at least one gene of a group of genes involved in the intracellular/extracellular transport function of the compound, a product thereof, or a combination thereof, wherein the gene or the product thereof is multi-componented.

2. The structural body according to claim 1, wherein the gene, a product thereof, or a combination thereof is labeled.

3. The structural body according to claim 1, wherein the group of genes is a group of genes involved in the regulation of the function or expression of an intracellular/extracellular transport function effector protein.

4. The structural body according to claim 3, wherein the transport is intracellular influx or extracellular efflux.

5. The structural body according to claim 1, wherein the product is a protein.

6. The structural body according to claim 5, wherein the protein is a soluble protein.

7. The structural body according to claim 5, wherein the protein has a specific expression form from among a plurality of expression forms.

8. A kit for analyzing a compound, the kit comprising the structural body according to claim 1.

9. A method for producing a structural body for capturing a compound, the method comprising the steps of: culturing a transformant containing an expression vector containing at least one gene of a group of genes involved in the intracellular/extracellular transport function of the compound; and collecting an expression product of said gene from the resulting culture product, wherein the gene is a multi-componented gene.

10. A method for binding a compound to the structural body according to claim 1, the method comprising a step of mixing the structural body with the compound.

11. A method for crystallizing the structural body according to claim 1, the method comprising a step of mixing the structural body with a solution for crystallization.

12. A method for analyzing a structural body crystallized by the method according to claim 11, the method comprising a step of analyzing the structure of the structural body by molecular replacement method.

Fig. 1

370 x 220 x 130 $\mu m^3$

Fig. 2

350 x 180 x 150 $\mu m^3$

Fig. 3

A

B

10

101

102a
103a

102b
103b

102c
103c

Fig. 4

20

202a

202b

203    201

A          B

C          D

202c

202d

Fig. 5

Fig. 6

300 x 50 x 50 µm³

Fig. 7

## INTERNATIONAL SEARCH REPORT

| International application No. |
| --- |
| **PCT/JP2022/048103** |

**A. CLASSIFICATION OF SUBJECT MATTER**

*C12N 15/12*(2006.01)i; *C12N 15/31*(2006.01)i; *C12P 21/02*(2006.01)i; *C07K 14/195*(2006.01)i; *C07K 14/37*(2006.01)i; *C07K 14/415*(2006.01)i; *C07K 14/435*(2006.01)i

FI: C12N15/31; C07K14/195; C07K14/37; C07K14/415; C07K14/435; C12N15/12; C12P21/02

According to International Patent Classification (IPC) or to both national classification and IPC

**B. FIELDS SEARCHED**

Minimum documentation searched (classification system followed by classification symbols)

C12N15/12; C12N15/31; C12P21/02; C07K14/195; C07K14/37; C07K14/415; C07K14/435

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | MATSUMOTO, T. et. al. Development of a structure determination method using a multidrug-resistance regulator protein as a framework. Biochemical and Biophysical Research Communications. 2019, vol. 518, pp. 402-408 | 1-8, 10-12 |
| | abstract, p. 402, right column, line 14 to p. 404, right column, line 15 | |
| Y | | 1-12 |
| Y | YAMASAKI, S. et al. The crystal structure of multidrug-resistance regulator RamR with multiple drugs. Nature Communications. 2013, vol. 4, no. 2078 | 1-12 |
| | abstract, p. 2, left column, line 1 to p. 3, left column, line 4, p. 6, left column, lines 27-41 | |
| Y | 西野 邦彦, 多剤排出ポンプによる細菌機能制御, Yakugaku Zasshi. 2012, vol. 132, no. 1, pp. 45-50, (NISHINO, Kunihiko. Physiological role of bacterial multidrug efflux pumps.) | 1-12 |
| | p. 45, left column, line 1 to p. 46, left column, line 38, p. 47, left column, line 14 to p. 48, right column, line 4 | |

☑ Further documents are listed in the continuation of Box C.　　☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **20 February 2023** | **07 March 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| PCT/JP2022/048103 |

**C.    DOCUMENTS CONSIDERED TO BE RELEVANT**

| Category* | Citation of document, with indication, where appropriate, of the relevant passages | Relevant to claim No. |
|---|---|---|
| Y | 西野 邦彦 他, グラム陰性菌における薬剤排出システムの役割, 日本化学療法学会雑誌, 2008, vol. 56, no. 4, pp. 443-452, (NISHINO, Kunihiko et al. Physiological functions of drug efflux systems in Gram-negative bacteria: Their roles in bacterial drug resistance and virulence. Japanese Journal of Chemotherapy.)<br>abstract, table 1, fig. 4 | 1-12 |
| Y | 吉田 博明, 細菌におけるキノロン耐性メカニズム, 日本細菌学雑誌, 1996, vol. 51, no. 4, pp. 973-992, (YOSHIDA, Hiroaki. Mechanisms of bacterial resistance to quinolones. Japanese Journal of Bacteriology.)<br>abstract, p. 974, left column, line 2 to p. 976, left column, line 20, fig. 1 | 1-12 |

Form PCT/ISA/210 (second sheet) (January 2015)

22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011062260 A
- WO 2014038220 A
- WO 2014038221 A

**Non-patent literature cited in the description**

- **TAKASHI MATSUMOTO et al.** *Biochemical and Biophysical Research Communications,* 2019, vol. 518, 402-408
- **SAMBROOK J. et al.** Molecular Cloning, A Laboratory Manual. Cold Spring Harbor Laboratory Press, 2012